# EUROPEAN PATENT APPLICATION

(11) **EP 3 632 296 A1**
(43) Date of publication of application: **08.04.2020**
(21) Application number: 19800541.5
(22) Date of filing: 25.04.2019
(51) Int. Cl.: A61B 1/12, A61B 1/00, B08B 9/027, G02B 23/24

(54) **CLEANING SYSTEM, CLEANING UNIT, AND CLEANING METHOD**

(30) Priority: 08.05.2018 JP 2018089683
(71) Applicant: Takashin Co., Ltd., Hirakawa-shi, Aomori 036-0114 (JP)
(72) Inventor: HIGUCHI Hirokazu, Kyoto-shi Kyoto 607-8308 (JP); TESHIMA Hidehiko, Nishio-shi, Aichi 445-0872 (JP); OMIYA Yoshitaka, Nagoya-shi, Aichi 463-0053 (JP); ISHIKAWA Hiroki, Tokyo 178-0064 (JP); FUJITA Masaki, Hirakawa-shi, Aomori 036-0115 (JP); KOGAWA Jun, Aomori 038-1211 (JP); SASAKI Tomiya, Hirosaki-shi, Aomori 036-8052 (JP); MIKAMI Hiroaki, Hirakawa-shi, Aomori 036-0242 (JP); NAKAMURA Eiichiro, Aomori-shi, Aomori 038-1305 (JP)
(74) Representative: Haseltine Lake Kempner LLP
(86) International application number: PCT/JP2019/017702
(87) International publication number: WO 2019/216246

(57) **Abstract**

The present invention provides a cleaning system in which a cleaning force is improved, a cleaning unit and a cleaning method. The cleaning system encompasses a tubular solution reservoir to reserve a pH-adjusted solution, defining a hollow space at an upper space on the pH-adjusted solution, a gas-filling path connected to a top of the solution reservoir, to send a carbon dioxide gas in the hollow space at a constant adjustment pressure, a carbon dioxide gas-cylinder connected to an input side of the gas-filling path, a solution-sending path connected to a lower part of the solution reservoir, to send the pH-adjusted solution to a pipeline of a Device-to-be-Cleaned at the adjustment pressure, a cleaning-solution introduction-path connected to the solution reservoir, to introduce an original cleaning solution to the solution reservoir, for generating the pH-adjusted solution, a stock-solution tank provided at an input side of the cleaning-solution introduction-path, configured to reserve the original cleaning solution, and a filling-pressure adjuster provided in the gas-filling path and a solution-sending-pressure adjuster provided in the solution-sending path, to obtain the adjustment pressure, by adjusting the filling pressure of the carbon dioxide gas-cylinder with the filling-pressure adjuster and the solution-sending-pressure adjuster.

## Description

### [Technical Field]

The present invention relates to a cleaning system for cleaning an inside of a pipeline of a tubular Device-to-be-Cleaned in which an inner diameter of the pipeline is three millimeters or less and its length is long as compared with the inner diameter, such as an endoscope, an endoscope treatment tool or the like, a cleaning unit used in the cleaning system, and a cleaning method of a Device-to-be-Cleaned using the cleaning system.

### [Background Art]

With regard to an endoscope commonly used in medicine, not only its outside but also its inside is required to be quickly cleaned and disinfected for each treatment. If cleaning and disinfection are insufficient, the fear of infection disease is increased, and an accident example is actually reported. The above is similarly pointed out for the cleaning and disinfection of the Device-to-be-Cleaned in various endoscope treatment tools, etc., such as a biological forceps, a high frequency snare, a contrast tube, etc.

A brushing method using a dedicated brush is recently recommended for a pre-disinfection cleaning of the inside of the pipeline in a endoscope. The endoscope is roughly classified into a flexible endoscope and a rigid endoscope. In "the flexible endoscope", there are a fiber scope in which an image captured by a lens system at a tip is guided through a glass fiber to an eyepiece outside a body and observed with naked eyes, and an electronic endoscope in which an image is transcribed to a solid-state imaging device at a tip, electrically guided to a monitor and observed. A device generally referred to as "a gastroscope" corresponds to the flexible endoscope. On the contrary, 'the rigid endoscope' has a simple structure in which lenses are attached to both ends of a cylinder, and an image is guided through a lens system and observed at the eyepiece outside the body. A laparoscope used in a laparoscope surgery is the rigid endoscope. Even each of a cystoscope and a thoracoscope corresponds to the rigid endoscope. In particular, the pipeline of the flexible endoscope is small in diameter and complicatedly bent. Thus, there was a problem that not only the cleaning of the pipeline by human hands took time and labor, but also uniformity could not be ensured in the quality after the cleaning. Since an endoscope with a wire had narrow pipeline, it became more difficult to clean.

Patent literature (PTL) 1 describes an invention of an endoscope cleaning device that can perform a cleaning action easily and surely in a short time. In the invention described in PTL 1, dirty substances, etc., adhered to the inner surface of the pipeline are removed by the high-speed jet flow of the gas-liquid two-phase foaming fluid in which carbon dioxide (CO₂) gas is mixed in a cleaning solution. Moreover, the removal effectiveness of the dirty substances is made higher by shock waves when many bubbles included in the foaming fluid are burst.

However, in the invention described in PTL 1, the foaming fluid is only pushed out into the pipeline of the endoscope by the initial pressure of the carbon dioxide gas itself within the cleaning device. Thus, the sending pressure of the fluid into the pipeline of the endoscope is not always as desired. The sending pressure is changed depending on the balance between a gas volume of a carbon dioxide gas-cylinder and a volume of a cleaning solution, and a kind of a cleaning solution, and the like. Also, from just after the start of the jet flow to its end, in a usual case, the sending pressure is only decreased. Thus, there is a problem that a constant cleaning force cannot be obtained because a constant sending pressure cannot be kept.

Also, in the invention described in PTL 1, a high fluid pressure of about 0.8MPa is applied as an initial value. A pressure that can be applied to a wire channel of a flexible endoscope is defined as a maximum of about 0.5MPa. Thus, if the invention described in PTL 1 is used, a risk that the flexible endoscope is broken is very high. A fiber-type flexible endoscope which is often used for cranial nerves and children is thinner than that of a usual endoscope for alimentary canal. Thus, the handling of the cleaning demands a great deal of care. Moreover, in recent years, an endoscope having a minimal inner diameter has been developed. For example, there is a type of a cholangioscope lifting from the tip of a duodenoscope whose inner diameter is about 0.5 millimeter. Hence, naturally, it must be handled more delicately.

Moreover, in the invention described in PTL 1, since the sending pressure (inner pressure) is not constant, a carbon dioxide gas's solubility to a cleaning solution is not constant too. Thus, it is difficult to manage pH level of the original cleaning solution. In a case of a cleaning solution that is usually weakly alkaline, oily stains and protein stains are decomposed by the decomposition force resulting from the above weak alkaline. Thus, if the dissolution of the carbon dioxide gas is proceeded and the pH level is lowered too much and biased to be acidic, a problem even as a cleaning function comes out. Also, there is a fear that the acidic solution deteriorates a rubber part of a component in the endoscope.

### [Citation List]

### [Patent Literature]

[PTL 1] JP 1987-34458A

### [Summary of Invention]

### [Technical Problem]

The present invention is made by paying attention to the above problems, and its object inheres in a cleaning system for improving a cleaning force to clean a tubular Device-to-be-Cleaned having a thin pipeline with an inner diameter of three millimeters or less, a cleaning unit used in the cleaning system, and a cleaning method of a Device-to-be-Cleaned using the cleaning system.

### [Solution to Problem]

In order to achieve the above object, a first aspect of the present invention inheres in a cleaning system encompassing (a) a tubular solution reservoir configured to reserve a pH-adjusted solution, defining a hollow space at an upper space on the pH-adjusted solution, (b) a gas-filling path connected to a top of the solution reservoir, configured to send a carbon dioxide gas in the hollow space at a constant adjustment pressure, (c) a carbon dioxide gas-cylinder connected to an input side of the gas-filling path, (d) a solution-sending path connected to a lower part of the solution reservoir, configured to send the pH-adjusted solution to a pipeline of a Device-to-be-Cleaned at the adjustment pressure, (e) a cleaning-solution introduction-path connected to the solution reservoir, configured to introduce an original cleaning solution to the solution reservoir, for generating the pH-adjusted solution, (f) a stock-solution tank provided at an input side of the cleaning-solution introduction-path, configured to reserve the original cleaning solution, and (g) a filling-pressure adjuster provided in the gas-filling path and a solution-sending-pressure adjuster provided in the solution-sending path, configured to obtain the adjustment pressure, by adjusting a filling pressure of the carbon dioxide gas-cylinder with the filling-pressure adjuster and the solution-sending-pressure adjuster. And, in the cleaning system pertaining to the first aspect, the pipeline is cleaned by the pH-adjusted solution.

A second aspect of the present invention inheres in a cleaning unit encompassing (a) a tubular solution reservoir configured to reserve a pH-adjusted solution, defining a hollow space at an upper space on the pH-adjusted solution, (b) a gas-filling path connected to a top of the solution reservoir, configured to send a carbon dioxide gas in the hollow space at a constant adjustment pressure, (c) a solution-sending path connected to a lower part of the solution reservoir, configured to send the pH-adjusted solution to a pipeline of a Device-to-be-Cleaned at the adjustment pressure, and (d) a filling-pressure adjuster provided in the gas-filling path and a solution-sending-pressure adjuster provided in the solution-sending path, configured to obtain the adjustment pressure, by adjusting a filling pressure of a carbon dioxide gas-cylinder with the filling-pressure adjuster and the solution-sending-pressure adjuster. And in the cleaning unit pertaining to the second aspect, the pipeline is cleaned by the pH-adjusted solution.

A third aspect of the present invention inheres in a cleaning method including (a) reserving an original cleaning solution in a tubular solution reservoir, (b) adjusting pH of the original cleaning solution, by filling a carbon dioxide gas into a hollow space defined on the original cleaning solution in an inside of the solution reservoir so as to generate a pH-adjusted solution, (c) sending a certain amount of the pH-adjusted solution reserved in the solution reservoir to a pipeline of a Device-to-be-Cleaned, by a pressure of the carbon dioxide gas filled in the hollow space, and (d) complementing a wasted amount of the original cleaning solution to the solution reservoir. In the cleaning method pertaining to the third aspect, the pipeline is cleaned by sequentially repeating processing loops, each of the loops encompassing the adjusting pH, the sending of the pH-adjusted solution, the complementing of the wasted amount, and returning to the adjusting pH.

### [Advantageous Effects of Invention]

According to the present invention, it is possible to provide the cleaning system for improving the cleaning force to clean the tubular Device-to-be-Cleaned, the cleaning unit used in the cleaning system, and the cleaning method of the Device-to-be-Cleaned using the cleaning system.

### [Brief Description of Drawings]

FIG. 1 is a schematic view of a cleaning system pertaining to a representative embodiment of the present invention;
FIG. 2 is a left perspective view of an example of a cleaning unit pertaining to the embodiment;
FIG. 3 is a left side view of the example of the cleaning unit pertaining to the embodiment;
FIG. 4 is a right perspective view of the example of the cleaning unit pertaining to the embodiment;
FIG. 5 is a right side view of the example of the cleaning unit pertaining to the embodiment;
FIG. 6 is a view illustrating a use situation of the cleaning unit pertaining to the embodiment; and
FIG. 7 is a schematic view of a cleaning system pertaining to a variation of the embodiment.

### [Description of Embodiments]

A representative embodiment (hereinafter called the "embodiment") and a variation of the embodiment of the present invention will be described below with reference to the drawings. Then, an improved cleaning system for cleaning a pipeline of a tubular Device-to-be-Cleaned in which an inner diameter is three millimeters or less and its length is long such as an endoscope or endoscope treatment tool or the like, a cleaning unit used in the cleaning system, and a cleaning method of a Device-to-be-Cleaned using the cleaning system are described. In the descriptions of the following drawings, the same or similar symbols are assigned to the same or similar portions. However, the drawings are merely diagrammatic, and attention should be paid to a fact that the relations between thicknesses and planar dimensions, and the ratio of the sizes of respective members and the like differ from the actual data. Thus, the specific thicknesses, dimensions sizes and the like should be judged more variously in light of the meaning of technical ideas that can be understood from the following explanations. Also, it is natural that even between the mutual drawings, the portions in which the relations and ratios between the mutual dimensions are different are included.

Also, the embodiment and the variation of the embodiment as described below exemplify the devices, etc., and methods for implementing the technical ideas of the present invention, and the technical ideas of the present invention are not narrowly limited to the endoscopes and endoscope treatment tools and the like as exemplified below, and the materials, shapes, structures, arrangements, etc., of their configuration parts are not specified to the followings. The technical ideas of the present invention are not limited to the contents described in the embodiment and the variation of the embodiment. Thus, various changes can be added to the technical ideas of the present invention within the technical scope defined by the claims.

### (Outline of Cleaning System)

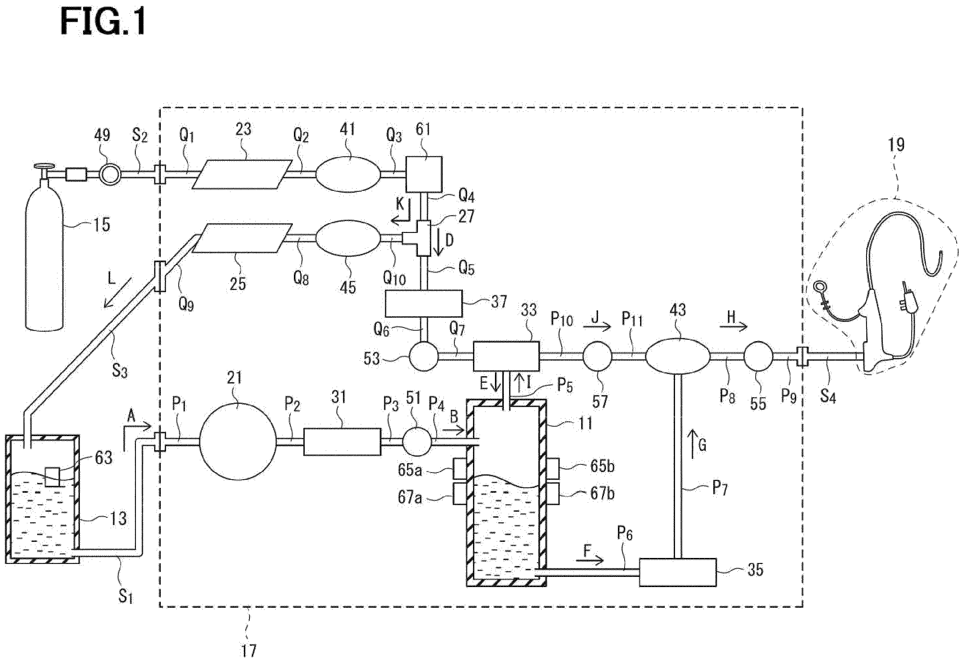

As illustrated schematically in FIG. 1, a cleaning unit 17 used in a cleaning system pertaining to the embodiment of the present invention includes a tubular solution reservoir 11 in which a pH-adjusted solution whose pH is adjusted is reserved and an upper space of the pH-adjusted solution is a hollow space, a gas-filling path (Q₁, 23, Q₂, 41, Q₃, 61, Q₄, 27, Q₅, 37, Q₆, 53, Q₇, 33 and P₅) which is connected to a top of the solution reservoir 11 and fills a carbon dioxide gas in the hollow space at a constant adjustment pressure, and a solution-sending path (P₆, 35, P₇, 43, P₈, 55 and P₉) which is connected to a lower part of the solution reservoir 11 and sends the pH-adjusted solution to a pipeline of an endoscope 19 connected to the cleaning unit 17, at the constant adjustment pressure. Here, as illustrated in FIG. 1, "the gas-filling path" includes a gas pipe Q₁, a first safety-valve 23, a gas pipe Q₂, a filling-pressure adjuster 41, a gas pipe Q₃, a pressure gauge 61, a gas pipe Q₄, a joint 27, a gas pipe Q₅, a gas-filling valve 37, a gas pipe Q₆, a second check-valve 53, a gas pipe Q₇, a top valve 33 and a liquid pipe P₅. Also, 'the solution-sending path' includes a liquid pipe P₆, an emission valve 35, a liquid pipe P₇, a solution-sending-pressure adjuster 43, a liquid pipe P₈, a third check-valve 55 and a liquid pipe P₉. "The constant adjustment pressure" when solution is sent to the pipeline of the endoscope 19 is obtained by adjusting a filling pressure of a carbon dioxide gas-cylinder 15 or a filling pressure of a cylinder in which pressure is decreased through a cylinder-side pressure-adjuster 49, by using the filling-pressure adjuster 41 installed in the gas-filling path (Q₁, 23, Q₂, 41, Q₃, 61, Q₄, 27, Q₅, 37, Q₆, 53, Q₇, 33 and P₅) and the solution-sending-pressure adjuster 43 installed in the solution-sending path (P₆, 35, P₇, 43, P₈, 55 and P₉). The cleaning unit 17 pertaining to the embodiment cleans the pipeline of the endoscope 19 by sending the pH-adjusted solution reserved in the solution reservoir 11 to the endoscope 19. FIG. 1 illustrates a flexible endoscope, as an example of the endoscope 19, which may serve as a cleaning target or "a Device-to-be-Cleaned". However, the endoscope 19 may be a rigid endoscope. Also, various treatment tools used in relation to an endoscope surgery can be used as the Device-to-be-Cleaned.

The cleaning unit 17 includes an entrance valve 31 connected to an upper side of the solution reservoir 11 and a sending pump 21 connected to the entrance valve 31. The sending pump 21 is connected to a stock-solution tank 13 and implements the cleaning system pertaining to the embodiment, and an original cleaning solution within the stock-solution tank 13 can be reserved in the inside of the solution reservoir 11 through the sending pump 21 and the entrance valve 31. An upper limit-sensor and a lower limit-sensor are attached to the solution reservoir 11. The upper limit-sensor is implemented by an upper light-emitter 65a and an upper light-receiver 65b in FIG. 1, and the lower limit-sensor is implemented by a lower light-emitter 67a and a lower light-receiver 67b in FIG. 1. Each of the above sensors is a sensor for detecting a water level of the inside of the solution reservoir 11. In FIG. 1, each of the upper and lower limit-sensors that are attached to the solution reservoir 11 is a transmission photoelectric sensor. However, if a water level detection function can be fulfilled, any of a photoelectric sensor with reflection or retro-reflective architecture, a capacitive sensor, a float sensor and the like is available.

"A connection" of each device, such as the solution reservoir 11 or the like, in this specification and the like may be either a direct connection or an indirect connection, if not particularly defined. A direct connection means a scheme in which respective devices are connected to each other so that they are in physical contact with each other or a scheme in which the respective devices are merely connected to each other through a pipe and the like. An indirect connection means that the respective devices are connected to each other through a different device other than a mere pipe. Also, a connected state means a situation in which liquid or gas or gas-liquid mixture or the like can be passed between the respective devices in one-direction or both-directions, regardless of whether direct or indirect, without leaking to the outside.

In the schematic view illustrated in FIG. 1, the inside of the solution reservoir 11 means the inside of a rectangle representing the cross-section of the solution reservoir 11. A wave line in the inside of the solution reservoir 11 represents a level of a water surface of liquid reserved in the solution reservoir 11. The lower side of the inside of the rectangle representing the cross-section of the solution reservoir 11 corresponds to "a bottom surface" of the solution reservoir 11. FIG. 1 represents a state in which the liquid is reserved from the bottom surface to the wave line. On the other hand, the upper side of the inside of the rectangle representing the cross-section of the solution reservoir 11 is "a ceiling surface" of the solution reservoir 11. In FIG. 1, a portion from the ceiling surface to the wave line corresponds to the hollow space and a state in which the hollow space is filled with gas is described. The stock-solution tank 13 in FIG. 1 has a similar configuration.

As illustrated schematically in FIG. 1, in the cleaning unit 17, the gas-filling path (Q₁, 23, Q₂, 41, Q₃, 61, Q₄, 27, Q₅, 37, Q₆, 53, Q₇, 33 and P₅) is connected to the carbon dioxide gas-cylinder 15 and implements the cleaning system pertaining to the embodiment. The carbon dioxide gas within the carbon dioxide gas-cylinder 15 can be filled in the hollow space of the solution reservoir 11 through the gas-filling path (Q₁, 23, Q₂, 41, Q₃, 61, Q₄, 27, Q₅, 37, Q₆, 53, Q₇, 33 and P₅).

As illustrated schematically in FIG. 1, in the cleaning unit 17 pertaining to the embodiment, the solution-sending path (P₆, 35, P₇, 43, P₈, 55 and P₉) is connected to the endoscope 19 illustrated at the right end in FIG. 1. That is, the pH-adjusted solution whose pH is adjusted in the solution reservoir 11 in the cleaning unit 17 is sent through the solution-sending path (P₆, 35, P₇, 43, P₈, 55 and P₉) to the endoscope 19 connected to the right end of the cleaning unit 17. Also, the liquid or gas or gas-liquid mixture or foamed fluid or the like in the solution reservoir 11 can be emitted through the path including the top valve 33 and the solution-sending-pressure adjuster 43 to outside the system of the cleaning unit 17. In a case that the liquid, etc., are evacuated to outside the system of the cleaning unit 17 except during the cleaning of the endoscope 19, it is preferred that the endoscope 19 is not connected to the liquid pipe P₉.

As illustrated schematically in FIG. 1, in the cleaning unit 17, the stock-solution tank 13 is connected to the sending pump 21, and the carbon dioxide gas-cylinder 15 is connected to the gas-filling path (Q₁, 23, Q₂, 41, Q₃, 61, Q₄, 27, Q₅, 37, Q₆, 53, Q₇, 33 and P₅), and the cleaning system pertaining to the embodiment is accordingly implemented, and an output pipeline to the endoscope 19 is connected to the solution-sending path (P₆, 35, P₇, 43, P₈, 55 and P₉). In the cleaning system pertaining to the embodiment, the original cleaning solution in the stock-solution tank 13 is reserved in the inside of the solution reservoir 11, and the carbon dioxide gas in the carbon dioxide gas-cylinder 15 is filled in the hollow space of the solution reservoir 11, and the pH-adjusted solution is generated. "The pH-adjusted solution" in this specification indicates a cleaning solution whose pH is changed by bringing the original cleaning solution and the carbon dioxide gas into contact with each other in some way. As a method of bringing them into contact, a method of merely bringing the liquid surface of the original cleaning solution and the carbon dioxide gas into contact with each other may be used, or a method of bubbling the carbon dioxide gas into the original cleaning solution and accordingly dissolving the carbon dioxide gas may be used, or the other methods may be used. A contact time is not mattered, and a value of adjusted pH is not mattered. When the endoscope 19 is cleaned, the pH-adjusted solution reserved in the inside of the solution reservoir 11 is sent through the solution-sending path, which includes the liquid pipe P₆, the emission valve 35, the liquid pipe P₇, the solution-sending-pressure adjuster 43, the liquid pipe P₈, the third check-valve 55 and the liquid pipe P₉, to the pipeline of the endoscope 19, at the constant pressure.

### (Detail of Cleaning Unit)

In FIG. 1, a first check-valve 51 is arranged between the solution reservoir 11 and the entrance valve 31. The upper part of the solution reservoir 11 is connected through the liquid pipe P₄ to the first check-valve 51, and the first check-valve 51 is connected through the liquid pipe P₃ to the entrance valve 31. The entrance valve 31 is connected through the liquid pipe P₂ to the sending pump 21, and the sending pump 21 is connected to the liquid pipe P₁, and the liquid pipe P₁ is connected to an external connection-port. On the other hand, the bottom part of the stock-solution tank 13 is connected to an external pipe (tube) S₁, and the external pipe S₁ is connected to the external connection-port of the liquid pipe P₁ in the cleaning unit 17. In short, after the original cleaning solution within the stock-solution tank 13 is passed through the external pipe S₁ of the bottom part of the stock-solution tank 13, the original cleaning solution is passed through a cleaning-solution introduction-path, which is implemented by the sending pump 21, the entrance valve 31, the first check-valve 51 and each liquid pipe Pᵢ (i = an integer of one to four), in A and B directions in FIG. 1, and reserved in the inside of the solution reservoir 11.

In FIG. 1, the top in the solution reservoir 11 is connected through the liquid pipe P₅ to the top valve 33. The second check-valve 53 is arranged between the top valve 33 and the gas-filling valve 37. The top valve 33 is connected through the gas pipe Q₇ to the second check-valve 53, and the second check-valve 53 is connected through the gas pipe Q₆ to the gas-filling valve 37. The joint 27 and the pressure gauge 61 are arranged between the gas-filling valve 37 and the filling-pressure adjuster 41. The gas-filling valve 37 is connected through the gas pipe Q₅ to the joint 27, and the joint 27 is connected through the gas pipe Q₄ to the pressure gauge 61. The pressure gauge 61 is connected through the gas pipe Q₃ to the filling-pressure adjuster 41.

The filling-pressure adjuster 41 is connected through the gas pipe Q₂ to a first safety-valve 23, and the first safety-valve 23 is connected through the gas pipe Q₁ to an external connection-port. At the external connection-port, the gas pipe Q₁ is connected to an external pipe (tube) S₂, and the external pipe S₂ is connected through the cylinder-side pressure-adjuster 49 to the carbon dioxide gas-cylinder 15. In short, the carbon dioxide gas in the carbon dioxide gas-cylinder 15 is passed through the cylinder-side pressure-adjuster 49 and the external pipe S₂, and passed through the gas-filling path, which is implemented by the gas pipe Q₁, the first safety-valve 23, the gas pipe Q₂, the filling-pressure adjuster 41, the gas pipe Q₃, the pressure gauge 61, the gas pipe Q₄, the joint 27, the gas pipe Q₅, the gas-filling valve 37, the gas pipe Q₆, the second check-valve 53, the gas pipe Q₇, the top valve 33 and the liquid pipe P₆, in C, D and E-directions in FIG. 1, and filled in the hollow space of the solution reservoir 11.

The top valve 33 is a 3-way solenoid valve for making the path switching of triple ports, and plays the roles of the supply of the carbon dioxide gas in the E-direction to the top of the solution reservoir 11 from the carbon dioxide gas-cylinder 15 (the gas-filling path), and the release of the gas and the like in the solution reservoir 11 to atmosphere in an I-direction (the pressure-reducing adjustment-path). With the path switching of the top valve 33, the liquid or gas or gas-liquid mixture or foamed fluid is passed to one of the E-direction and the I-direction within the liquid pipe P₅.

A gas-pressure reducer 45 is connected through the gas pipe Q₁₀ to the joint 27, and a second safety-valve 25 is connected through the gas pipe Qs to the gas-pressure reducer 45. The gas-pressure reducer 45 and the second safety-valve 25 implement a very low-pressure gas-sending mechanism. The second safety-valve 25 is connected to the gas pipe Q₉, and the gas pipe Q₉ is connected to an external connection-port. At the external connection-port, the gas pipe Q₉ is connected to an external (tube) pipe S₃, and the external pipe S₃ is connected to the top of the stock-solution tank 13. A part of the carbon dioxide gas emitted from the carbon dioxide gas-cylinder 15 is flowed to a K-direction at the joint 27, and passed through a flow path, which is implemented by the gas-pressure reducer 45 and the second safety-valve 25 and each gas pipe Qⱼ (j = an integer of eight to ten) and passed through the external pipe S₃ in an L-direction in FIG. 1 and sent to the hollow space of the stock-solution tank 13.

In FIG. 1, the lower end (bottom part) of the solution reservoir 11 is connected through the liquid pipe P₆ to the emission valve 35, and the emission valve 35 is connected through the liquid pipe P₇ to the solution-sending-pressure adjuster 43. The third check-valve 55 is arranged between the solution-sending-pressure adjuster 43 and the endoscope 19, and a fourth check-valve 57 is arranged between the solution-sending-pressure adjuster 43 and the top valve 33. The solution-sending-pressure adjuster 43 is connected through the liquid pipe P₈ to the third check-valve 55, and the third check-valve 55 is connected to the liquid pipe P₉, and the liquid pipe P₉ is connected to an external connection-port. At the external connection-port, the liquid pipe P₉ is connected to an external pipe (tube) S₄, and the external pipe S₄ is connected to the endoscope 19. The solution-sending-pressure adjuster 43 is connected through the liquid pipe P₁₁ to the fourth check-valve 57, and the fourth check-valve 57 is connected through the liquid pipe P₁₀ to the top valve 33. Although illustration is omitted, a flow meter for measuring a solution-sending amount may be installed at any location of the solution-sending path (P₆, 35, P₇, 43, P₈, 55 and P₉).

At a time of filling the carbon dioxide gas, when the gas-filling valve 37 is opened, the carbon dioxide gas emitted from the carbon dioxide gas-cylinder 15 is flowed through the gas-filling path of the top valve 33 set in the E-direction in FIG. 1 to the solution reservoir 11. In the phase of sending the pH-adjusted solution (pressure feeding) to the endoscope 19, the gas-filling valve 37 is closed by electric signals. And, the emission valve 35 is opened by electric signals, and the pH-adjusted solution in the solution reservoir 11 is passed through the solution-sending path, which is implemented by the emission valve 35, the solution-sending-pressure adjuster 43, the third check-valve 55 and each liquid pipe Pᵢ (i = an integer of six to nine) in F, G and H-directions in FIG. 1 and sent through the external pipe S₄ to the pipeline of the endoscope 19.

On the other hand, at a time of a pressure-reducing adjustment in which the gas pressure in the solution reservoir 11 is reduced, as follows, the switching to the pressure-reducing adjustment-path is carried out because the top valve 33 is implemented by the 3-way valve. Thus, the emission valve 35 is closed, the liquid or gas or gas-liquid mixture or foamed fluid or the like in the solution reservoir 11 is passed through the pressure-reducing adjustment-path, which is implemented by the top valve 33, the fourth check-valve 57, the solution-sending-pressure adjuster 43, the third check-valve 55 and each liquid pipe Pᵢ (i = an integer of five and eight to eleven), in the I, J and H-directions in FIG. 1 and exhausted to outside the system of the cleaning unit 17, and the gas in the solution reservoir 11 is released to atmosphere, and the pressure-reducing adjustment is accordingly performed.

Although the check-valves such as the first check-valve 51 and the like in FIG. 1 are not essential, they are preferably provided from the viewpoint of preventing the backflow for the their main purpose and avoiding the risk such as any failure of the other devices and the like. Even the safety-valves such as the first safety-valve 23 and the like in FIG. 1 are similar.

The sending pump 21 in FIG. 1 may be a diaphragm pump. However, if a water sending force can be ensured, it is possible to use the pump of a different type. For example, it is possible to variously apply a turbo pump represented by a centrifugal pump, a vane pump, a gear pump and the like. However, from the viewpoint of ensuring the quantitative property of the solution-sending action, it is preferred to apply a positive displacement pump such as a diaphragm pump.

A water level sensor 63 of a float type is installed in the inside of the stock-solution tank 13 in FIG. 1. When the amount of the original cleaning solution in the stock-solution tank 13 becomes lower than a certain amount, a signal is emitted, which stops the solution-sending process of the cleaning unit 17. When the water level in the stock-solution tank 13 is raised again, the solution-sending process is started. For the water level sensor 63, it is possible to use a sensor of a different type, such as a photoelectric sensor, a capacitive sensor and the like, in addition to the float sensor, if the water level detection function can be fulfilled.

In FIG. 1, the materials, shapes and arrangement of the various pipes, such as the liquid pipe P₁ and the like, are not limited to the particular ones. Any one may be used as long as it is chemically and physically resistant to the solution properties and sending pressures of the original cleaning solution, the pH-adjusted solution and the carbon dioxide gas.

### (Preparation Phase)

The operation of the cleaning system pertaining to the embodiment is described with reference to FIG. 1. Before entering the preparation phase as the first step, the cleaning unit 17 is supposed to be connected to the stock-solution tank 13 and the carbon dioxide gas-cylinder 15. At the preparation phase, at first, in order to make the gas pressure within the path of the cleaning unit 17 similar to an atmosphere pressure, the 3-way valve serving as the top valve 33 is switched for establishing a preparation-pressure adjusting-path. "The preparation-pressure adjusting-path" is a path through which the original cleaning solution is sent from the stock-solution tank 13 to the solution reservoir 11, in a valve state in which the pressure-reducing adjustment-path as mentioned above is set, when the entrance valve 31 is opened and the sending pump 21 is operated. When the water level of the original cleaning solution arrives at the upper limit-sensor (65a and 65b), the entrance valve 31 is closed and the top valve 33 is switched to the gas-filling path. In a case that the pH-adjusted solution remains in the solution reservoir 11 at the initial timing of the preparation phase, when the gas pressure within the solution reservoir 11 is suddenly reduced to the atmosphere pressure, the carbon dioxide gas dissolved in the remain of the pH-adjusted solution is released, and foaming is generated in the pH-adjusted solution. There is a fear that the generated foam disturbs the sensor functions of the water level sensors (the upper and lower limit-sensors) of the solution reservoir 11. Thus, for emitting the foam to outside the system of the cleaning unit 17, the excess amount of the original cleaning solution is flowed. After the correct inflow of the original cleaning solution to the solution reservoir 11 is checked, the endoscope 19 is connected to the cleaning unit 17, as illustrated in FIG. 1.

### (pH-Adjusting Phase)

At the pH-adjusting phase as the second step, the gas-filling valve 37 is opened by electric signals, and the filling of the carbon dioxide gas from the carbon dioxide gas-cylinder 15 is started. At this time, in order to prevent flowing the carbon dioxide gas whose pressure is higher than 0.5MPa , the pressure-reducing adjustment is performed on the first safety-valve 23. After that, in the filling-pressure adjuster 41, the gas pressure is adjusted to 0.45MPa. In the pressure gauge 61, when the first safety-valve 23 or the filling-pressure adjuster 41 are troubled by any chance, the gas sending process and the water sending process are stopped in a case of detecting the inflow of high pressure gas. The carbon dioxide gas correctly passed to the pressure gauge 61 is branched into two paths at the joint 27. A part of the carbon dioxide gas is flowed to the D-direction, passed through the gas-filling valve 37, flowed through the top valve 33 which is switched to the gas-filling path in the E-direction, and filled in the hollow space of the solution reservoir 11, and the pH-adjusted solution is accordingly adjusted (generated). At this time, the entrance valve 31 and the emission valve 35 are closed, and the first check-valve 51 and the second check-valve 53 make closed space. Thus, the inner pressure of the carbon dioxide gas is made high in the hollow space of the solution reservoir 11. The other part of the carbon dioxide gas is flowed in the K-direction, and its pressure is reduced to about 100 to 101KPa close to the atmosphere pressure by the gas-pressure reducer 45 implementing the very low-pressure gas-sending mechanism. In the second safety-valve 25 implementing the very low-pressure gas-sending mechanism as with the gas-pressure reducer 45, only the pressure exceeding 100KPa, namely, the carbon dioxide gas having a very low pressure less than 1KPa is sent to the hollow space of the stock-solution tank 13. That is, the above is the mechanism in which with regard to the carbon dioxide gas, when some extent of the original cleaning solution is flowed out from the stock-solution tank 13, the carbon dioxide gas of the very low pressure whose volume is corresponding to an outflow amount, is supplied to the stock-solution tank 13.

### (Solution-sending Phase)

At the solution-sending phase as the third step, the gas-filling valve 37 is closed by electric signals, and the emission valve 35 is opened by electric signals. Then, the pH-adjusted solution-sending to the pipeline of the endoscope 19 is started. At the solution-sending phase, the solution-sending-pressure adjuster 43 adjusts the sending pressure of the pH-adjusted solution to 0.45MPa. The third check-valve 55 is a check-valve for the sake of an infectious disease countermeasure and prevents the contaminated gas-liquid from being back-flowed from the side of the endoscope 19.

### (Complement Phase)

At a complement phase as the fourth step, when the water level of the pH-adjusted solution within the solution reservoir 11 is detected by the lower limit-sensor (67a and 67b), the emission valve 35 is closed by electric signals, and the entrance valve 31 is opened by electric signals. Then, the sending pump 21 is operated, and until the water level detected by the upper limit-sensor, the original cleaning solution is complemented to the pH-adjusted solution within the solution reservoir 11. When the water level is detected by the upper limit-sensor (65a and 65b), the entrance valve 31 is closed by electric signals.

### (Processing Loop)

When the complement phase has been completed, the operational flow is again returned to the pH-adjusting phase as the second step, and proceeds through the solution-sending phase as the third step to the complement phase as the fourth step. Namely, the pipeline of the endoscope 19 is cleaned by repeating the processing loop sequentially several times in which the operational flow proceeds from the pH-adjusting phase as the second step through the solution-sending phase as the the third step and the complement phase as the fourth step and returned to the pH-adjusting phase as the second step. The number of the processing loops required for one endoscope cleaning may be directly controlled on the basis of the number of the reactions of the upper limit-sensor (65a and 65b) and the lower limit-sensor (67a and 67b) in FIG. 1. Or, if the flow meter is installed in the solution-sending path in the cleaning system, the above number of the processing loops may be controlled by repeating the processes in the processing loop until the solution-sending amount exceeds the set total amount of the pH-adjusted solution. Also, if it is possible to grasp the solution-sending amount of the pH-adjusted solution per unit time, the operation of the cleaning system may be controlled by setting the operational time of the cleaning system.

With regard to the solution-sending amount of the pH-adjusted solution at one solution-sending phase, any amount of about 0.95 × 10⁻⁶ m³ (0.95 mL) or more can be set by adjusting an interval between the upper and lower limit-sensors. However, the amount is preferably set at 3 to 8 × 10⁻⁶ m³. The smaller amount of the solution to be sent at one time is preferable for the sake of the stabilization of the water level within the solution reservoir 11. However, since the amount of the original cleaning solution that is complemented to the solution reservoir 11 at one time is smaller, a contact time with the carbon dioxide gas becomes longer, and the rate of a contact area becomes larger. Accordingly, the pH level of the pH-adjusted solution within the solution reservoir 11 is reduced to near a neutral (between 7.0 and 7.5). When the solubility of the carbon dioxide gas to the original cleaning solution is high, it is possible to improve the cleaning force resulting from the dissolved carbon dioxide gas. However, on the other hand, the pH level of the original cleaning solution is about 10, namely, weakly alkaline. Thus, even the pH level of the pH-adjusted solution containing the carbon dioxide gas is desired to hold the range of weak alkali (over 8.0 and below 11.0).

The sending pressure of the carbon dioxide gas, the gas sending method, the sending pressure of the pH-adjusted solution, the mixing method of the original cleaning solution and the carbon dioxide gas, the size of the solution reservoir 11 and the like were totally reviewed with various experiments. As a result, it was known that preferably, the pH level of the pH-adjusted solution in which the cleaning force of the pipeline of the endoscope 19 was most improved was around 9, and the sending pressure of the pH-adjusted solution including the carbon dioxide gas was between 0.4 and 0.45 MPa, and more preferably, the sending pressure was around 0.45 MPa. When the pH is reduced below about 9, the cleaning force is decreased. Also, when the sending pressure is between 0.2 and 0.3 MPa, the cleaning force is low, and when the sending pressure is higher than 0.45 MPa, there is a fear that the structure of the endoscope 19 is broken. In order to realize the pH and the sending pressure of the pH-adjusted solution suitable for cleaning the endoscope, as the size of the solution reservoir 11, its inner diameter is preferably ten millimeters or more, and the preferable material of the solution reservoir 11 may be urethane group substances, which are excellent in durability. It is not preferable that the inner diameter of the solution reservoir 11 is less than ten millimeters, because, when any bubbles are generated within the pH-adjusted solution, it is difficult to remove the bubbles from the pH-adjusted solution. When the tubular solution reservoir 11 of the size whose inner diameter was ten millimeters or more was used, as the solution-sending amount of the pH-adjusted solution at one solution-sending phase and the compensate amount of the original cleaning solution at one compensate phase, 3 to 8 × 10⁻⁶m³ was an appropriate amount.

It is preferable not to install a mixture mechanism of the carbon dioxide gas and the original cleaning solution. The pH levels desirable for the pH-adjusted solution and the cleaning force resulting from the pH can be obtained by a method of bringing the carbon dioxide gas into simple contact with the liquid surface of the original cleaning solution, at the constant pressure. Thus, the further installation of the mixture mechanism is not preferable because the dissolution of the carbon dioxide gas is progressed more than necessary.

For the original cleaning solution that is used in the cleaning system pertaining to the embodiment, medical detergent of weak alkaline and non-enzymatic group based on natural ingredients is used. However, the original cleaning solution is not limited to the above medical detergent. However, the filths targeted for cleaning are the contaminants of organic components such as proteins, fats, oils and the like. Thus, the weak alkaline or alkaline is preferable in order to dissolve those contaminants.

As mentioned above, according to the cleaning system pertaining to the embodiment, by compressively sending the pH-adjusted solution containing the carbon dioxide gas, the pH-adjusted solution is kept weak alkaline, to the pipeline of the Device-to-be-Cleaned, the synergistic effect of the cleaning force can be achieved. Namely, the synergistic effect is ascribable to a cleaning force resulting from the bubbles of the carbon dioxide gas, and another cleaning force resulting from the liquid property of the weak alkaline originally possessed by the pH-adjusted solution. Then, the synergistic effect enables the higher cleaning force to be obtained as compared with the conventional cleaning method. Also, the process for compressively sending the pH-adjusted solution at a high pressure (about 0.45 MPa) at which the structure of the Device-to-be-Cleaned is not broken contributes to the high cleaning force. It is very difficult to clean a tubular Device-to-be-Cleaned which has a long length and small diameter pipeline, for example the pipeline may have an inner diameter of three millimeters or less. Particularly, the inner diameter of the pipeline may be two millimeters or less, and further the inner diameter of the pipeline may be one millimeter or less. However, according to the cleaning system pertaining to the embodiment, it is possible to sufficiently clean the long and small diameter pipeline as mentioned above. In particular, it is possible to sufficiently clean even an endoscope whose inner diameter is about 0.5 millimeter, such as a cholangioscope lifting from the tip of a duodenoscope. Among the endoscope treatment tools, the tools having a tubular shape, such as a spraying tube, a contrast tube and the like, can be cleaned by connecting the cleaning system pertaining to the embodiment to the pipeline of the above tools. Among the endoscope treatment tools, the tools having a non-tubular shape, such as a biological forceps, a high frequency snare and the like, can be cleaned by pouring the pH-adjusted solution which is compressively sent by the cleaning system, to the endoscope treatment tools instead of directly connecting to the cleaning system pertaining to the embodiment. The endoscope targeted for cleaning does not matter whether the endoscope is flexible or rigid. Also, even the rigid endoscope for a surgery robot and treatment tools of the surgery robot are similarly targeted for cleaning.

In particular, in the cleaning system pertaining to the embodiment, at the pH-adjusting phase, the filling pressure of the carbon dioxide gas from the carbon dioxide gas-cylinder 15 is adjusted to the gas pressure of 0.45 MPa by using the filling-pressure adjuster 41 and the solution-sending-pressure adjuster 43, and the pH-adjusted solution being kept at weak alkaline is compressively sent to the pipeline of the Device-to-be-Cleaned. For this reason, the cleaning unit 17 pertaining to the embodiment does not require an additional solution-sending device, such as a pump and the like, for sending the pH-adjusted solution to the pipeline of the Device-to-be-Cleaned. That is, the cleaning unit 17 pertaining to the embodiment does not require the solution-sending device for compressively sending the pH-adjusted solution to the pipeline of the Device-to-be-Cleaned. Thus, the configuration of the cleaning system can be made small and light, and its installation area can be small. Hence, even the tubular Device-to-be-Cleaned which has the long length and small diameter pipeline having the inner diameter of three millimeters or less, particularly the inner diameter of two millimeters or less, and further the inner diameter of one millimeter or less, can be cleaned by using the compact cleaning unit 17.

Also, the operation and architecture of the cleaning unit 17 pertaining to the embodiment is automated although its configuration is compact. Thus, the result of the cleaning is uniform, and its labor and time are less. Hence, this is a system in which the consumption amounts of the carbon dioxide gas and the original cleaning solution are fewer and its cost is lower, as compared with a case conducted by a human hand.

Moreover, according to the cleaning system pertaining to the embodiment, the solution-sending amount of the pH-adjusted solution at one solution-sending phase is small, for example 3 to 8 ×10⁻⁶ m³. Thus, it is possible to decrease the volume occupation rate of the solution reservoir occupying the entire system. Hence, there is a merit that the miniaturization of the entire system can be effectively realized by taking the advantage that any solution-sending devices such as a pump and the like are not required as mentioned above.

Moreover, according to the cleaning system pertaining to the embodiment, the pH-adjusted solution in which the carbon dioxide gas is dissolved has the foamability, and bubbling is conventionally problematic. However, by providing a mechanism for putting the generated bubbles to outside the system of the cleaning unit 17, it is possible to avoid the malfunction of the water level sensor caused by the bubble generation.

Moreover, according to the cleaning system pertaining to the embodiment, a mechanism in which, in response to the outflow-volume amount of the original cleaning solution, the carbon dioxide gas is filled into the stock-solution tank is provided, which enables the inside of the stock-solution tank to be kept cleaner without being exposed to the dirty outside air. The carbon dioxide gas to be filled is extremely low in pressure. Thus, the pH level of the pH-adjusted solution is never dropped more than necessary.

### (Example of Cleaning Unit)

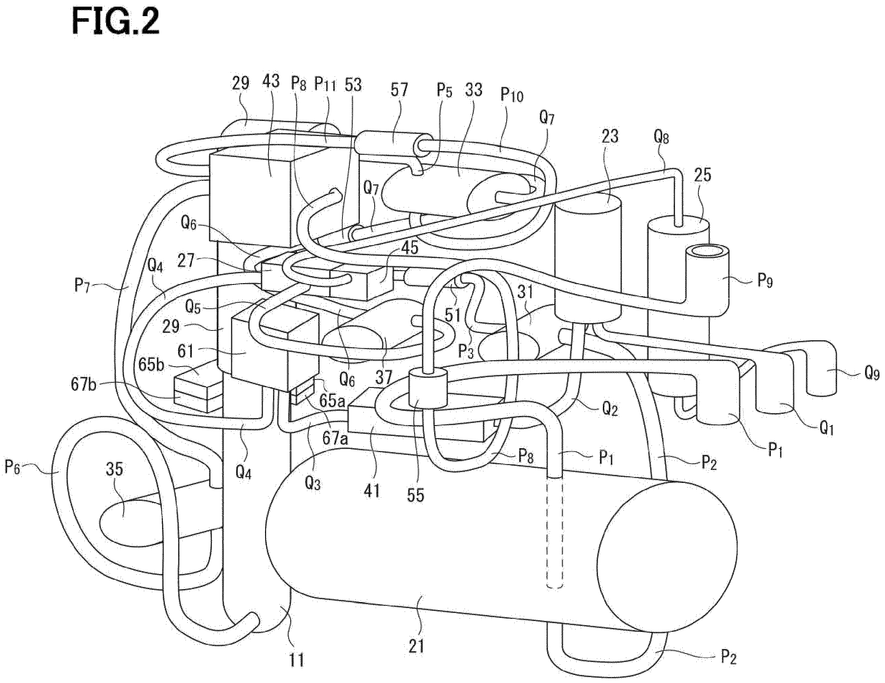

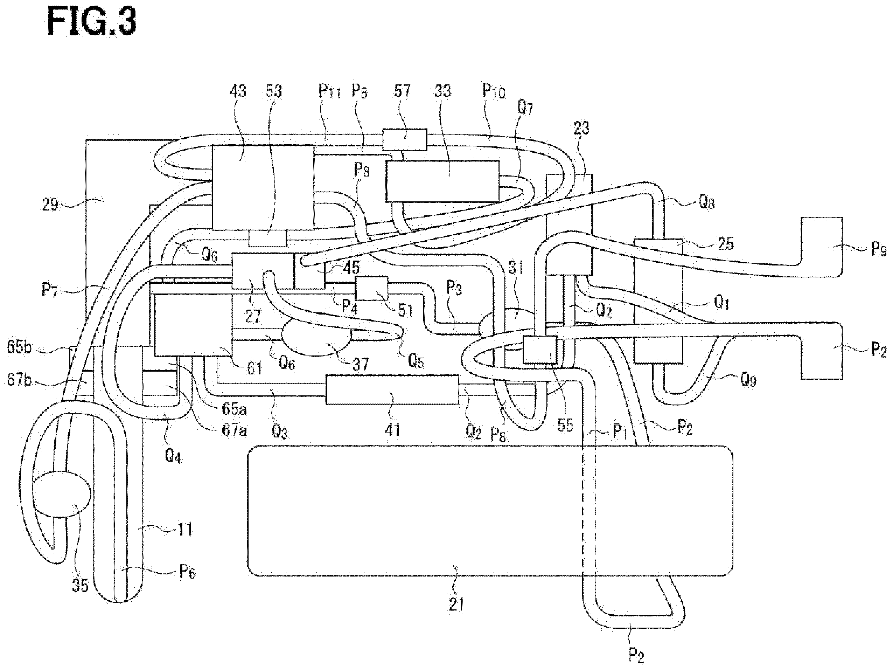

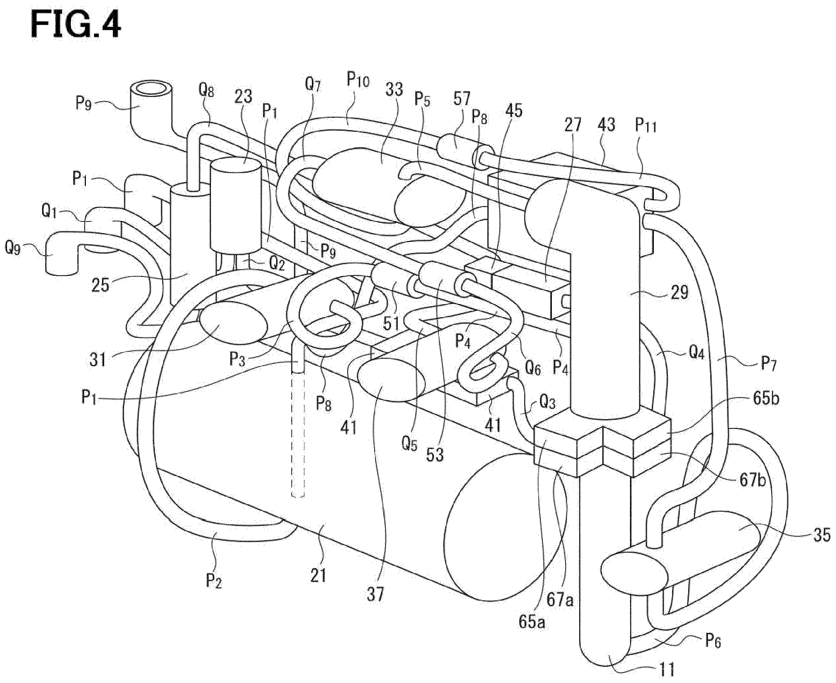

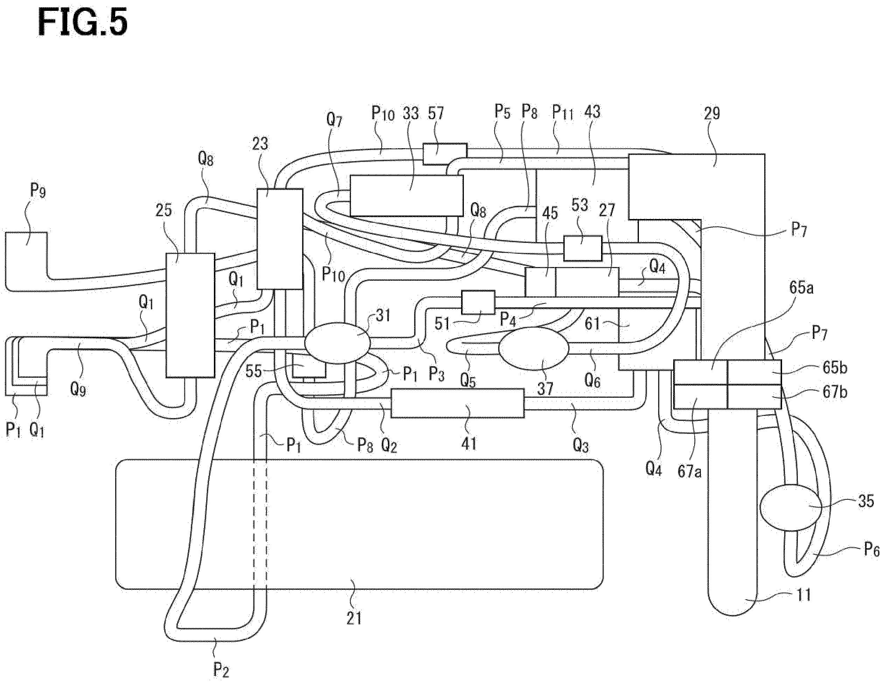

The cleaning unit implementing the cleaning system pertaining to the embodiment can be stored in an enclosing case and used in an easy-handling condition. The arrangement of respective elements of the cleaning unit pertaining to the embodiment, in a case when the enclosing case or the outer enclosure is removed, is illustrated in FIGs. 2, 3, 4 and 5. Each of FIGs. 2, 3, 4 and 5 is a view from a different angle of the inside of the enclosing case of a cleaning machine for cleaning the Device-to-be-Cleaned, such as an endoscope or the like, which includes the cleaning unit pertaining to the embodiment.

### (Configuration of Example of Cleaning Unit)

As illustrated in FIGs. 2, 3, 4 and 5, the upper space of the tubular solution reservoir 11 having the hollow space is connected to a gas-liquid injector 29. In the solution reservoir 11, the upper and lower limit-sensors are attached to a side of the solution reservoir 11. The upper limit-sensor is implemented by the upper light-emitter 65a and the upper light-receiver 65b in FIG. 2, etc. The lower limit-sensor is implemented by the lower light-emitter 67a and the lower light-receiver 67b in FIG. 2, etc. All of them detect water levels of the inside of the solution reservoir 11. The first check-valve 51 is arranged between the gas-liquid injector 29 and the entrance valve 31. The gas-liquid injector 29 is connected through the liquid pipe P₄ to the first check-valve 51, and the first check-valve 51 is connected through the liquid pipe P₃ to the entrance valve 31. The entrance valve 31 is connected through the liquid pipe P₂ to the sending pump 21, and the sending pump 21 is connected through the liquid pipe P₁ to a stock-solution tank. Accordingly, it is possible to implement the cleaning system pertaining to the embodiment. In short, an original cleaning solution within a stock-solution tank is passed through the flow path (the cleaning-solution introduction-path), which is implemented by the sending pump 21, the entrance valve 31, the first check-valve 51, the gas-liquid injector 29 and each liquid pipe Pᵢ (i = an integer of one to four) and reserved in the inside of the solution reservoir 11 and adjusted to a pH-adjusted solution.

In FIG. 2, etc., the gas-liquid injector 29 is connected through the liquid pipe P₅ to the top valve 33. The second check-valve 53 is arranged between the top valve 33 and the gas-filling valve 37. The top valve 33 is connected through the gas pipe Q₇ to the second check-valve 53, and the second check-valve 53 is connected through the gas pipe Q₆ to the gas-filling valve 37. The joint 27 and the pressure gauge 61 are arranged between the gas-filling valve 37 and the filling-pressure adjuster 41. The gas-filling valve 37 is connected through the gas pipe Q₅ to the joint 27, and the joint 27 is connected through the gas pipe Q₄ to the pressure gauge 61, and the pressure gauge 61 is connected through the gas pipe Q₃ to the filling-pressure adjuster 41.

To the filling-pressure adjuster 41, the first safety-valve 23 is connected through the gas pipe Q₂. The first safety-valve 23 is connected through the gas pipe Q₁ to a carbon dioxide gas-cylinder. Accordingly, it is possible to implement the cleaning system pertaining to the embodiment. In short, a carbon dioxide gas within a carbon dioxide gas-cylinder is passed through a flow path (gas-filling path), which is implemented by the first safety-valve 23, the filling-pressure adjuster 41, the pressure gauge 61, the joint 27, the gas-filling valve 37, the second check-valve 53, the top valve 33, the gas-liquid injector 29, each gas pipe Qⱼ (j = an integer of one to seven) and the liquid pipe P₅, and filled into the hollow space of the solution reservoir 11.

The gas-pressure reducer 45 is connected to the joint 27, and the second safety-valve 25 is connected through the gas pipe Q₈ to the gas-pressure reducer 45. The second safety-valve 25 is connected through the gas pipe Q₉ to a stock-solution tank. Accordingly, it is possible to implement the cleaning system pertaining to the embodiment. A part of a carbon dioxide gas emitted from a carbon dioxide gas-cylinder is flowed to the gas-pressure reducer 45 at the joint 27, and passed through the flow path, which is implemented by the gas-pressure reducer 45, the second safety-valve 25 and each gas pipe Qⱼ (j = eight and nine), and sent to the hollow space of a stock-solution tank.

In FIG. 2, etc., the lower part of the solution reservoir 11 is connected through the liquid pipe P₆ to the emission valve 35, and the emission valve 35 is connected through the liquid pipe P₇ to the solution-sending-pressure adjuster 43. The solution-sending-pressure adjuster 43 is connected through the liquid pipe P₈ to the third check-valve 55, and the third check-valve 55 can be connected through the liquid pipe P₉ to a pipeline of an endoscope. The solution-sending-pressure adjuster 43 is connected through the liquid pipe P₁₁ to the fourth check-valve 57, and the fourth check-valve 57 is connected through the liquid pipe P₁₀ to the top valve 33.

At a time of an usual operation, the top valve 33 is set to the gas-filling path, and when the gas-filling valve 37 is opened, a carbon dioxide gas emitted from a carbon dioxide gas-cylinder is flowed to the solution reservoir 11. When the pH-adjusted solution is compressively sent to an endoscope, the gas-filling valve 37 is closed by electric signals, and the emission valve 35 is opened by electric signals. Then, a pH-adjusted solution within the solution reservoir 11 is passed through the flow path (the solution-sending path), which is implemented by the emission valve 35, the solution-sending-pressure adjuster 43, the third check-valve 55 and each liquid pipe Pᵢ (i = an integer of six to nine), and flowed to a pipeline of an endoscope.

On the other hand, in a case that a gas pressure within the solution reservoir 11 is required to be dropped, the top valve 33 is switched to the pressure-reducing adjustment-path. The emission valve 35 is closed, and a liquid, a gas, a gas-liquid mixture, a foamed fluid or the like within the solution reservoir 11 is passed through the flow path (the pressure-reducing adjustment-path), which is implemented by the gas-liquid injector 29, the top valve 33, the fourth check-valve 57, the solution-sending-pressure adjuster 43, the third check-valve 55 and each liquid pipe Pᵢ (i = an integer of five and eight to eleven), and emitted to outside the system of the cleaning unit pertaining to the embodiment. In this case, an endoscope is preferred to be a state, which is not to be connected to the liquid pipe P₉.

The functions and structures of the respective devices in the cleaning unit pertaining to the embodiment illustrated in FIG. 2, etc., and the using methods and effectiveness of the cleaning system and the like are similar to those of the cleaning unit 17 pertaining to the embodiment illustrated in FIG. 1 and the cleaning system implemented by the cleaning unit 17. In the cleaning unit pertaining to the embodiment illustrated in FIG. 2, etc., since the respective pipelines are intricately interlaced and the respective devices are accordingly arranged, there is a merit that it is possible to realize the miniaturization in the whole of the cleaning system pertaining to the embodiment including the cleaning unit and it is also possible to achieve the space saving.

### (Use State of Cleaning System)

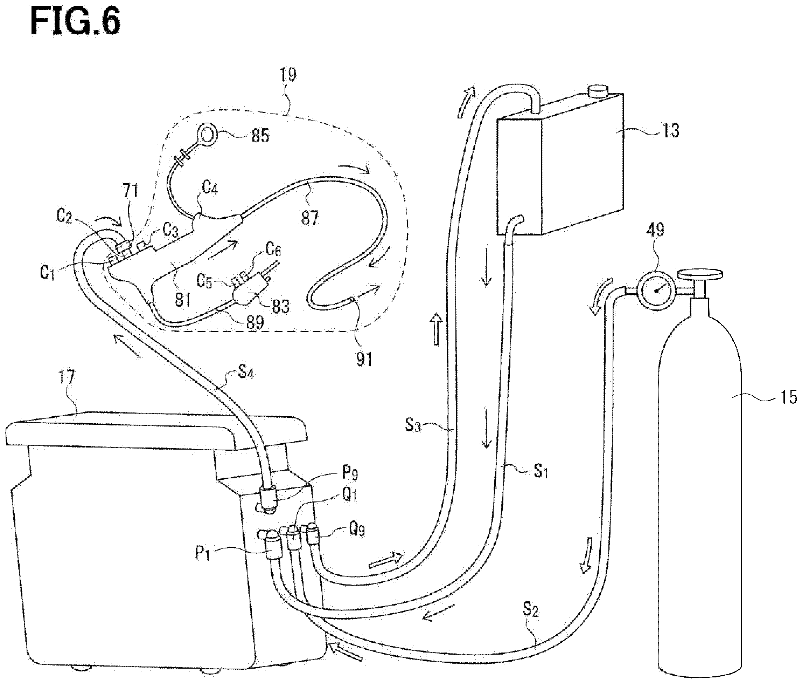

As illustrated in FIG. 6, the cleaning unit 17 implementing the cleaning system pertaining to the embodiment can be used, in a configuration such that the cleaning unit 17 is connected to the stock-solution tank 13, to the carbon dioxide gas-cylinder 15, and to the endoscope 19 through external pipes (tubes) Sk (k = an integer of one to four). The end of the liquid pipe P₁ of the cleaning unit 17 is connected through the external pipe S₁ to the stock-solution tank 13. The end of the gas pipe Q₁ is connected through the external pipe S₂ to the carbon dioxide gas-cylinder 15, and the end of the gas pipe Q₉ is connected through the external pipe S₃ to the stock-solution tank 13, and the end of the liquid pipe P₉ is connected through the external pipe S₄ to the endoscope 19.

As illustrated by arrow marks in FIG. 6, the original cleaning solution within the stock-solution tank 13 is passed through the external pipe S₁ and sent from the liquid pipe P₁ to the cleaning unit 17. The carbon dioxide gas within the carbon dioxide gas-cylinder 15 is passed through the external pipe S₂ and sent from the gas pipe Q₁ to the cleaning unit 17. A part of the carbon dioxide gas sent to the cleaning unit 17 is sent from the gas pipe Q₉ through the external pipe S₃ to the stock-solution tank 13. The pH-adjusted solution including the carbon dioxide gas, which is adjusted within the cleaning unit 17, is compressively sent from the liquid pipe P₉ through the external pipe S₄ to the pipeline of the endoscope 19.

As illustrated in FIG. 6, a general main structure of the endoscope 19 has an operation unit 81 serving as a main unit for operation, a connector 83 to be connected to an external device, a universal-code portion 89 through which the operation unit 81 and the connector 83 are linked to each other, and an inserter 87 to be inserted to a human body. The operation unit 81 has a forceps port C₄ into which a forceps 85 is inserted, and a tip of the forceps 85 is pulled out from a tip 91 of the inserter 87, and various surgeries are accordingly performed. In addition, various pipelines from a wire channel C₁, a sucking channel C₂, a gas-water sending channel C₃, and channels C₅ and C₆ of the connector 83 are connected to the tip 91. Although illustration is omitted, there is an endoscope that has a water sending sub-channel for sending a sterile water, etc., to clean a diseased site and the like. In short, in the entire structure of the endoscope 19 implemented by the connector 83, the universal-code portion 89, the inserter 87, etc., an inside of the endoscope 19 exhibits the complicated pipeline. Output / input ports of pipelines are respectively assigned to channels Cₘ (m = an integer of one to six), and the external pipe S₄ can be connected through an adaptor 71 to each channel Cₘ, and the pipeline can be cleaned by the pH-adjusted solution compressively sent from the cleaning unit 17. In FIG. 6, the external pipe S₄ is connected through the adaptor 71 to the sucking channel C₂, and in accordance with the flow of the arrow marks, the compressively-sent pH-adjusted solution is flowed through the operation unit 81 and the pipeline of the inside of the inserter 87. And thereafter, the pH-adjusted solution is evacuated from the tip 91. The structure of the pipeline of the endoscope 19 illustrated in FIG. 6 illustrates an example of flexible endoscopes. Although the positions of the respective channels and the kinds of the pipelines are different in response to the kind of flexible endoscopes, it can be similarly cleaned by the cleaning system pertaining to the embodiment.

The structures and arrangements of the respective elements of the cleaning unit 17 pertaining to the embodiment illustrated in FIG. 6 are omitted. However, the functions and structures of the respective elements and the using methods and effectiveness of the cleaning system, etc., are similar to those of the cleaning unit 17 pertaining to the embodiment illustrated in FIG. 1 and the cleaning system implemented by the cleaning unit 17.

FIG. 6 illustrates the flexible endoscope as the endoscope 19 targeted for cleaning. However, the endoscope 19 may be a rigid endoscope, or one of various treatment tools related to an endoscope surgery can be used as a Device-to-be-Cleaned. For a rigid endoscope whose illustration is omitted, a laparoscope is representative, and there is an apparatus into which a treatment tool is inserted, or an apparatus that has a function for irrigating water. The rigid endoscope is used to enucleate a gallbladder in a case for digestive organ, and the rigid endoscope is used to enucleate a fibroid in a case for gynecology, and the rigid endoscope is used for a surgery for enucleating a transurethral prostate and a kidney in a case for urology. For a thin rigid endoscope, there is an apparatus for observing an eardrum, a nasal cavity and a vocal cord, etc., in a case for otolaryngology, and in a case for ophthalmology, an endoscope is used at surgeries of vitreous and glaucoma, for the observation of the back of iris, the removal of vitreous turbidity, and the coagulation of laser light and the like.

Although illustration is omitted, a non-contact switch for controlling the operation of the cleaning system may be installed on the upper surface or side surface of the enclosing case for storing the cleaning unit 17 that implements the cleaning system pertaining to the embodiment. As an example of the non-contact switch, a switch, which uses an infrared sensor that reacts when a part of a human body, such as a hand, an elbow or the like, is held at a constant distance for a constant time, may be installed. Also, a foot switch may be installed instead of the non-contact switch. In conventional conditions, for cleaning an endoscope, gloves are used to prevent the mutual contamination and infection between an used endoscope and a worker. However, when any switch is installed on the upper surface or side surface of the enclosing case of the conventional instrument, miscellaneous contaminations or infections to the cleaning unit or third parties through the switch are concerned. When the non-contact switch or the foot switch is installed for the operation of the cleaning system pertaining to the embodiment, it is possible to prevent the above contaminations and infections. Therefore, the cleaning system pertaining to the embodiment can be used in a clean condition.

### (CLEANING SYSTEM OF VARIATION)

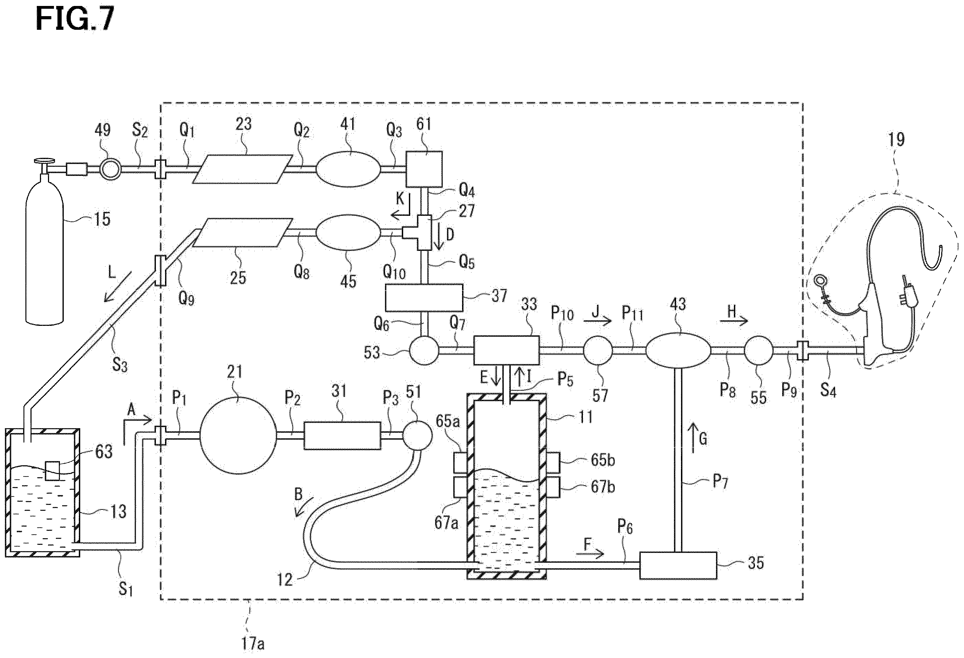

As illustrated schematically in FIG. 7, a configuration of a cleaning unit 17a used in a cleaning system pertaining to a variation of the embodiment differs from the cleaning unit 17 in FIG. 1 only in the connection locations and connection members between a solution reservoir 11 and a cleaning-solution introduction-path. That is, in the cleaning unit 17 in FIG. 1, the upper part of the solution reservoir 11 and the first check-valve 51 are connected to each other through the liquid pipe P₄. However, in the cleaning unit 17a in FIG. 7, a lower part of the solution reservoir 11 and a first check-valve 51 are connected to each other through a buffer tube 12. The other members implementing the cleaning unit 17a in FIG. 7 are similar to the members implementing the cleaning unit 17 in FIG. 1, and the schemes of the connections of the respective members are also similar. Thus, their explanations are omitted. Also, among the members and devices that implement the cleaning system pertaining to the variation illustrated in FIG. 7, even those other than the cleaning unit 17a are similar to the cleaning system pertaining to the embodiment illustrated in FIG. 1. The connection architectures and using methods of the above are also similar. The cleaning unit 17a in FIG. 7 can be stored in an enclosing case and used for cleaning the endoscope, similarly to the cleaning unit 17 in FIG. 1.

As to the buffer tube 12 of the cleaning unit 17a in FIG. 7, for example, its inner diameter can be four to six millimeters, and its length can be longer than all of liquid pipes P₁, P₂ and P₃, for example, 1500 to 3000 millimeters. For the length of the buffer tube 12, 1500 to 2000 millimeters is more preferable for the sake of reducing an installation space. The buffer tube 12 may be directly connected to the first check-valve 51 and the lower part of the solution reservoir 11 or may be indirectly connected by using an adaptor and the like. As illustrated in FIG. 7, the cleaning-solution introduction-path is implemented by a sending pump 21, an entrance valve 31, the first check-valve 51, respective liquid pipes Pᵢ (i = an integer of one to three) and the buffer tube 12. The buffer tube 12 is illustrated in an inverted S shape in FIG. 7. However, the topology of the buffer tube 12 is merely an exemplification. Thus, when the cleaning unit 17a pertaining to the variation is stored in an enclosing case, the buffer tube 12 may be folded several times. For folding the buffer tube 12 several times, the buffer tube 12 may be formed of a material that can be easily folded, for example, the buffer tube 12 may be formed of polyurethane and the like.

In a preparation phase of an operation of the cleaning system pertaining to the variation, similarly to the cleaning system pertaining to the embodiment, when the water level of the original cleaning solution arrives at an upper limit-sensor (65a and 65b), the entrance valve 31 is closed, and a top valve 33 is switched to a gas-filling path. At this time, since the cleaning-solution introduction-path is connected to the lower part of the solution reservoir 11, liquid splashes that are likely to occur when the original cleaning solution is introduced from the upper part are less likely to occur. Thus, the liquid surface within the solution reservoir 11 is stabilized, which can suppress the erroneous detection of the upper limit-sensor (65a and 65b). The complement phases of the original cleaning solutions are also similar. When the water level of the pH-adjusted solution within the solution reservoir 11 is detected by a lower limit-sensor (67a and 67b), the original cleaning solution is complemented to the pH-adjusted solution within the solution reservoir 11 until the water level detected by the upper limit-sensor (65a and 65b). Even at this time, the liquid splashes are less likely to occur. Thus, the liquid surface within the solution reservoir 11 is stabilized, which can suppress the erroneous detection of the upper limit-sensor (65a and 65b).

Moreover, in a flexible endoscope, each inner diameter of the gas-water sending channel and the sucking channel is larger than that of the wire channel, and an inner diameter of a pipeline of a rigid endoscope is larger than that of a flexible endoscope. When the gas-water sending channel, the sucking channel and the pipeline of the rigid endoscope are cleaned, a solution-sending amount is relatively increased as compared with a case of cleaning the wire channel whose inner diameter is smaller. Thus, the increased solution-sending amount will increase the driving frequency of the sending pump 21 in FIG. 7. As the frequency of the opening / closing of the entrance valve 31 located between the sending pump 21 and the solution reservoir 11 increases, a phenomenon in which water droplets are jumped upwardly from the liquid surface within the solution reservoir 11, ascribable to the impact (water hammer) resulting from the opening / closing, will easy occur. Thus, a frequency of the erroneous detection of the upper limit-sensor (65a and 65b) or the lower limit-sensor (67a and 67b) is also increased. In the cleaning unit 17a in FIG. 7, an impact is absorbed by the buffer tube 12 having the long length and small diameter pipeline, and the great impact is not transmitted to the pH-adjusted solution within the solution reservoir 11 at a time. Also, the phenomenon in which the water droplets are jumped upwardly from the liquid surface is hardly generated. Consequently, it is possible to prevent the erroneous detection of the upper limit-sensor (65a and 65b) or the lower limit-sensor (67a and 67b).

According to the cleaning system pertaining to the variation, by compressively sending the pH-adjusted solution containing the carbon dioxide gas, the pH-adjusted solution being kept alkaline, to the pipeline of the Device-to-be-Cleaned, the synergistic effect of a cleaning force due to the bubbles of the carbon dioxide gas and another cleaning force due to the liquid property of the weak alkaline, which is originally possessed by the pH-adjusted solution, can be achieved. Then, the synergistic effect enables the higher cleaning force to be obtained as compared with the conventional cleaning method. Also, the process for compressively sending the pH-adjusted solution at the high pressure (about 0.45 MPa) at which the structure of the Device-to-be-Cleaned is not broken contributes to the high cleaning force. It is very difficult to clean the tubular Device-to-be-Cleaned which has the long length and small diameter pipeline with an inner diameter of three millimeters or less, particularly the inner diameter of two millimeters or less, and further the inner diameter of one millimeter or less. However, according to the cleaning system pertaining to the variation, it is possible to sufficiently clean the above pipeline. In particular, it is possible to sufficiently clean even an apparatus whose inner diameter is about 0.5 millimeter, such as a cholangioscope. Among an endoscope treatment tools, the tools of the tubular shape, such as a spraying tube, a contrast tube and the like, can be cleaned by connecting the cleaning system pertaining to the variation to the pipelines of the above tools. Among endoscope treatment tools, the tools having the non-tubular shape, such as a biological forceps, a high frequency snare and the like, can be cleaned, without connecting directly to the cleaning system pertaining to the variation, by pouring the pH-adjusted solution, which is compressively sent by the cleaning system, to the endoscope treatment tools.

In particular, in the cleaning system pertaining to the variation, at a pH-adjusting phase, the filling pressure of the carbon dioxide gas from a carbon dioxide gas-cylinder 15 is adjusted to the gas pressure of 0.45 MPa by using a filling-pressure adjuster 41 and a solution-sending-pressure adjuster 43, and the pH-adjusted solution being kept at weak alkaline is compressively sent to the pipeline of the Device-to-be-Cleaned. For this reason, the cleaning unit 17a pertaining to the variation does not require an additional solution-sending device, such as a pump and the like, for sending the pH-adjusted solution to the pipeline of the Device-to-be-Cleaned. That is, the cleaning unit 17a pertaining to the variation does not require the solution-sending device for compressively sending the pH-adjusted solution to the pipeline of the Device-to-be-Cleaned. Thus, the configuration of the cleaning system can be made small and light, and its installation area can be small. Hence, even the tubular Device-to-be-Cleaned which has the long length and small diameter pipeline having the inner diameter of three millimeters or less, particularly the inner diameter of two millimeters or less, and further the inner diameter of one millimeter or less, can be cleaned by using the compact cleaning unit 17a.

Also, the operation and architecture of the cleaning unit 17a pertaining to the variation is automated although its configuration is compact. Thus, the result of the cleaning is uniform, and its labor and time are less. Hence, the cleaning unit 17a pertaining to the variation provides a system in which the consumption amounts of the carbon dioxide gas and the original cleaning solution are fewer and its cost is lower, as compared with the case conducted by a human hand.

Moreover, according to the cleaning system pertaining to the variation, the solution-sending amount of the pH-adjusted solution at one solution-sending phase is small, for example 3 to 8 × 10⁻⁶ m³. Thus, it is possible to decrease the volume occupation rate of the solution reservoir occupying the entire system. Hence, there is a merit that the miniaturization of the entire system can be effectively realized.

Conventionally, because the pH-adjusted solution, in which the carbon dioxide gas is dissolved, has the foamability, the bubbling behavior ascribable to the foamability is problematic. However, according to the cleaning system pertaining to the variation, by providing a mechanism for putting the generated bubbles to outside the system of the cleaning unit 17a, it is possible to avoid the malfunction of the water level sensor caused by the bubble generation.

Moreover, because the cleaning system pertaining to the variation has a mechanism in which, in response to the outflow-volume amount of the original cleaning solution, the necessary carbon dioxide gas is filled into the stock-solution tank, the inside of the stock-solution tank is kept cleaner without being exposed to the dirty outside air. Because the carbon dioxide gas to be filled is extremely low in pressure, the pH level of the pH-adjusted solution is never dropped more than necessary.

### (OTHER EMBODIMENT)

As mentioned above, the present invention are described by using the embodiment and the variation of the embodiment. However, the discussions and drawings that implement a part of this disclosure should not be construed to limit the present invention. From this disclosure, the various variation embodiments, implementations and operational techniques may be clear for a person skilled in the art.

Also, parts of the respective technical ideas explained in the embodiment and the variation of the embodiment can be properly combined with each other. In this way, naturally, the present invention includes various embodiments that are not described here. Thus, the technical scope of the present invention is determined only by the matters specifying the invention pertaining to the claims, which can be construed to be reasonable from the above explanations.

### [Reference Signs List]

- 11: a solution reservoir
- 12: a buffer tube
- 13: a stock-solution tank
- 15: a carbon dioxide gas-cylinder
- 17, 17a: a cleaning unit
- 19: an endoscope
- 21: a sending pump
- 23: a first safety-valve
- 25: a second safety-valve
- 27: a joint
- 29: a gas-liquid injector
- 31: an entrance valve
- 33: a top valve
- 35: an emission valve
- 37: a gas-filling valve
- 41: a filling-pressure adjuster
- 43: a solution-sending-pressure adjuster
- 45: a gas-pressure reducer
- 49: a cylinder-side pressure-adjuster
- 51: a first check-valve
- 53: a second check-valve
- 55: a third check-valve
- 57: a fourth check-valve
- 61: a pressure gauge
- 63: a water level sensor (of cleaning solution)
- 65a: an upper light-emitter
- 65b: an upper light-receiver
- 67a: a lower light-emitter
- 67b: a lower light-receiver
- 71: an adaptor
- 81: an operation unit (of endoscope)
- 83: a connector (of endoscope)
- 85: a forceps
- 87: an inserter (of endoscope)
- 89: an universal-cord portion (of endoscope)
- 91: a tip (of inserter of endoscope)
- P₁ to P₁₁: liquid pipes
- Q₁ to Q₁₀: gas pipes
- S₁ to S₄: external pipes
- C₁: a wire channel
- C₂: a sucking channel
- C₃: a gas-water sending channel
- C₄: a forceps port
- C₅: a channel (of endoscope)
- C₆: a channel (of endoscope)

## Claims

1. A cleaning system, comprising:
a tubular solution reservoir configured to reserve a pH-adjusted solution, defining a hollow space at an upper space on the pH-adjusted solution;
a gas-filling path connected to a top of the solution reservoir, configured to send a carbon dioxide gas in the hollow space at a constant adjustment pressure;
a carbon dioxide gas-cylinder connected to an input side of the gas-filling path;
a solution-sending path connected to a lower part of the solution reservoir, configured to send the pH-adjusted solution to a pipeline of a Device-to-be-Cleaned at the adjustment pressure;
a cleaning-solution introduction-path connected to the solution reservoir, configured to introduce an original cleaning solution to the solution reservoir, for generating the pH-adjusted solution;
a stock-solution tank provided at an input side of the cleaning-solution introduction-path, configured to reserve the original cleaning solution: and
a filling-pressure adjuster provided in the gas-filling path and a solution-sending-pressure adjuster provided in the solution-sending path, configured to obtain the adjustment pressure, by adjusting the filling pressure of the carbon dioxide gas-cylinder with the filling-pressure adjuster and the solution-sending-pressure adjuster,
wherein the pipeline is cleaned by the pH-adjusted solution.

2. The cleaning system of claim 1, wherein the cleaning-solution introduction-path is connected to a lower side of the solution reservoir.

3. The cleaning system of claim 2 or 3, further comprising:
an entrance valve provided at a part of the cleaning-solution introduction-path; and
a sending pump connected to the entrance valve, configured to implement the cleaning-solution introduction-path.

4. The cleaning system of claim 3, wherein the adjustment pressure is 0.45 MPa.

5. A cleaning unit, comprising:
a tubular solution reservoir configured to reserve a pH-adjusted solution, defining a hollow space at an upper space on the pH-adjusted solution;
a gas-filling path connected to a top of the solution reservoir, configured to send a carbon dioxide gas in the hollow space at a constant adjustment pressure;
a solution-sending path connected to a lower part of the solution reservoir, configured to send the pH-adjusted solution to a pipeline of a Device-to-be-Cleaned at the adjustment pressure; and
a filling-pressure adjuster provided in the gas-filling path and a solution-sending-pressure adjuster provided in the solution-sending path, configured to obtain the adjustment pressure, by adjusting a filling pressure of a carbon dioxide gas-cylinder with the filling-pressure adjuster and the solution-sending-pressure adjuster,
wherein the pipeline is cleaned by the pH-adjusted solution.

6. The cleaning unit of claim 5, further comprising a cleaning-solution introduction-path connected to the solution reservoir, configured to introduce an original cleaning solution to the solution reservoir, for generating the pH-adjusted solution.

7. The cleaning unit of claim 6, wherein the cleaning-solution introduction-path is connected to a lower side of the solution reservoir.

8. The cleaning unit of claim 6 or 7, further comprising:
an entrance valve provided at a part of the cleaning-solution introduction-path; and
a sending pump connected to the entrance valve, configured to implement the cleaning-solution introduction-path.

9. The cleaning unit of claim 8, further comprising upper and lower limit-sensors, attached to the solution reservoir,
wherein a complement amount of the original cleaning solution to the solution reservoir is controlled by signals from the upper and lower limit-sensors.

10. The cleaning unit of claim 9, wherein the adjustment pressure is 0.45 MPa.

11. A cleaning method including:
reserving an original cleaning solution in a tubular solution reservoir;
adjusting pH of the original cleaning solution, by filling a carbon dioxide gas into a hollow space defined on the original cleaning solution in an inside of the solution reservoir so as to generate a pH-adjusted solution;
sending a certain amount of the pH-adjusted solution reserved in the solution reservoir to a pipeline of a Device-to-be-Cleaned, by a pressure of the carbon dioxide gas filled in the hollow space; and
complementing a wasted amount of the original cleaning solution to the solution reservoir,
wherein the pipeline is cleaned by sequentially repeating processing loops, each of the loops comprising the adjusting pH, the sending of the pH-adjusted solution, the complementing of the wasted amount, and returning to the adjusting pH.
